# FASCICULE DE BREVET EUROPEEN

(11) **EP 1 343 419 B1**
(45) Date de publication et mention de la délivrance du brevet: **31.12.2008**
(21) Numéro de dépôt: 01997260.3
(22) Date de dépôt: 20.11.2001
(51) Int. Cl.: A61B 17/04, A61B 17/064, A61B 17/08

(54) **ATTACHE DE FIXATION PROTHETIQUE ET DISPOSITIF DE MISE EN PLACE DE CETTE ATTACHE**
PROTHETISCHE KLAMMER UND VORRICHTUNG ZUM EINSETZEN DIESER KLAMMER
PROSTHETIC FIXING FASTENER AND DEVICE FOR DELIVERING SAME

(30) Priorité: 24.11.2000 FR 0015239
(43) Date de publication de la demande: 17.09.2003
(73) Titulaire: SOFRADIM PRODUCTION, 01600 Trévoux (FR)
(72) Inventeur: SGRO, Jean-Claude, F-21000 DIJON (FR); THERIN, Michel, F-69004 LYON (FR); ORY, François-Régis, F-69270 FONTAINES SAINT MARTIN (FR); BAILLY, Pierre, 69300 Caluire (FR)
(74) Mandataire: Brédeville, Odile Marie
(86) Numéro de dépôt international: PCT/FR2001/003650
(87) Numéro de publication internationale: WO 2002/041790

(56) Documents cités:
- EP-A- 0 847 727
- WO-A-00/40159
- FR-A- 2 773 057
- FR-A- 2 774 277
- GB-A- 2 306 110
- US-A- 4 696 300
- US-A- 5 320 633

## Description

La présente invention concerne une attache de fixation prothétique, par exemple en matériau biorésorbable, utile pour fixer une pièce prothétique plane dans le corps humain par rapport à un support anatomique. Elle concerne également le dispositif de mise en place de cette attache.

Selon la présente invention, les termes utilisés ci-après dans la description et les revendications ont la signification suivante :
- par « *matériau biorésorbable* », on entend un matériau qui est décomposé et/ou assimilé par et/ou dans le corps humain ou animal ;
- par « *support anatomique »,* on entend des tissus humains ou animaux, de soutien, non minéralisés.

Il est connu par le document EP-A-0 847 727 une attache prothétique destinée à la réparation d'un ménisque fracturé. Cette attache est composée d'au moins un premier barreau d'arrêt destiné à venir en appui contre un fragment de ménisque, d'au moins un second barreau d'arrêt destiné à venir en appui contre un autre fragment de ménisque et d'une tige de liaison courbe, élastiquement déformable. Lesdits barreaux d'arrêt sont reliés de manière rigide à cette tige de liaison de telle sorte que chacun d'eux s'étend perpendiculairement à l'extrémité de la tige de liaison à laquelle il est relié.

Lorsque l'attache est implantée, la tige de liaison est déformée vers une configuration rectiligne de sorte qu'elle tend à rapprocher les barreaux d'arrêt l'un de l'autre et qu'elle assure ainsi le maintien des fragments de ménisques pressés l'un contre l'autre.

L'attache selon ce document convient pour réparer un ménisque mais ne pourrait être utilisée pour fixer une pièce prothétique plane, par exemple un renfort pariétal, à un support anatomique, en particulier une paroi abdominale. En effet, cette attache à tige de liaison élastiquement déformable et à barreaux d'arrêt reliés de manière rigide à cette tige de liaison interdit toute insertion dudit premier barreau d'arrêt à l'intérieur d'une paroi tissulaire, et ladite élasticité n'est pas adapté à un tel ancrage.

Le document WO 00/40159 décrit une attache de fixation également prévue pour réparer un ménisque et ne pouvant être utilisée pour fixer une pièce prothétique plane à un support anatomique.

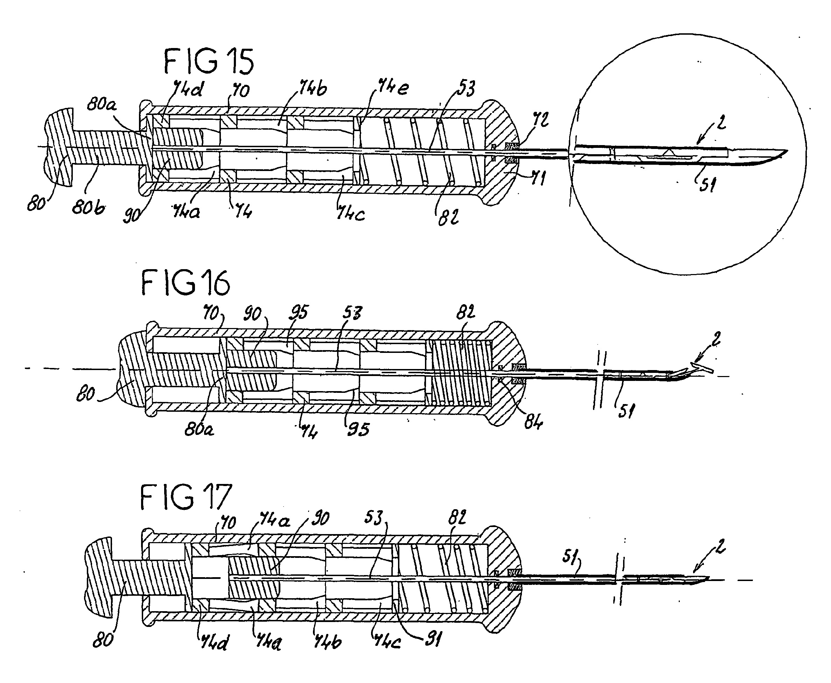

Le document US-C-5 203 864 décrit une attache de fixation en forme de "H" prévue pour fixer une pièce prothétique plane à un support anatomique (cf. figure 17). Cette attache comprend :
- un élément distal d'ancrage dans le support anatomique ;
- un élément proximal d'arrêt par rapport à la pièce prothétique ; et
- une tige de liaison reliant, d'un côté, l'élément distal d'ancrage, en un point intermédiaire de ce dernier, et, de l'autre côté, l'élément proximal d'arrêt, en un point intermédiaire de ce dernier.

L'attache selon ce document antérieur et le dispositif de mise en place de celle-ci ont pour inconvénient essentiel de ne pas assurer un contact intime de la pièce prothétique avec le support anatomique, alors qu'un tel contact intime est fondamental pour la bonne intégration tissulaire de ladite pièce prothétique. En effet, l'élément distal d'ancrage est déployé à une profondeur déterminée par la venue en butée de l'élément proximal d'arrêt contre la pièce prothétique ; or, compte tenu de la forme de l'attache, ce déploiement génère un certain recul de l'élément distal d'ancrage par rapport à cette profondeur, duquel il résulte le défaut précité de contact intime. De plus, la présence de la tige de liaison sur le côté de l'élément distal d'ancrage constitue une gêne à l'insertion de cet élément distal d'ancrage et peut provoquer une certaine lésion du site d'ancrage, ce qui a pour effet non seulement d'affecter la profondeur d'implantation de l'élément distal d'ancrage mais également la résistance de l'ancrage obtenu. Il peut également en résulter ledit défaut de contact intime.

La présente invention vise à solutionner ce défaut essentiel.

L'attache qu'elle concerne a, de manière connue en soi, une structure monolithique, susceptible d'être obtenue par moulage d'un matériau plastique, par exemple biorésorbable, et est composée d'au moins un élément d'ancrage dans un support anatomique, d'au moins un élément d'arrêt par rapport à une pièce prothétique plane à fixer, et d'une tige de liaison disposée entre une zone de jonction avec l'élément d'ancrage et une zone de jonction avec l'élément d'arrêt.

Selon l'invention, la tige de liaison est rectiligne et les zones de jonction de l'élément d'ancrage et de l'élément d'arrêt avec cette tige de liaison permettent à l'attache de prendre au moins trois conformations distinctes, à savoir :
- une conformation de démoulage, non contrainte, dans laquelle les directions respectives de l'élément d'arrêt et de l'élément d'ancrage forment entre elles, par exemple dans un même plan comprenant la tige de liaison, un angle intérieur au moins égal à 90° ;
- une conformation à contrainte maximum, ramassée sur elle-même, par exemple tubulaire par insertion complète de ladite attache dans un tube, dans laquelle l'élément d'ancrage et l'élément d'arrêt sont repliés vers la tige de liaison et l'un vers l'autre, quasiment bout à bout ; et
- une conformation à contrainte minimum, dans laquelle les directions respectives de l'élément d'arrêt et de l'élément d'ancrage forment entre elles, par exemple dans un même plan, un angle au plus égal à 90°.

L'attache selon l'invention permet un abord chirurgical totalement différent de celui utilisé traditionnellement. L'abord traditionnel consiste, comme cela est décrit dans le document US-C-5 203 864, à accéder, par l'intérieur d'une cavité anatomique, à la face postérieure d'une paroi tissulaire et à y fixer la ou les attaches requises pour le maintien ou la fixation de la pièce prothétique plane.

Grâce à l'invention, l'attache peut être complètement insérée dans ledit tube, avec ledit élément d'arrêt tourné vers l'extrémité distale de ce tube et ledit élément d'ancrage tourné vers l'extrémité proximale de ce tube. Ce dernier forme une aiguille creuse d'implantation permettant, depuis la face antérieure du support anatomique, d'insérer l'attache au-delà de la pièce prothétique située contre la face postérieure de ce support ; une extraction partielle de l'attache hors du tube permet de libérer ledit élément d'arrêt, qui peut prendre appui contre la pièce prothétique, puis un mouvement de recul du tube permet de libérer le reste de l'attache et d'implanter ainsi l'élément d'ancrage à une profondeur assurant le contact intime de la pièce prothétique avec le support anatomique.

Le dispositif de mise en place de l'attache comprend par conséquent, selon l'invention, ledit le tube et l'attache précités, celle-ci étant insérée dans ce tube avec ledit élément d'arrêt tourné vers l'extrémité distale de ce tube et ledit élément d'ancrage tourné vers l'extrémité proximale de ce tube ; ce tube comporte une extrémité distale acérée, présentant une ouverture latérale, et est pourvu d'un moyen de rétention de l'élément d'ancrage en deçà d'un seuil de force ; l'ouverture latérale est dimensionnée de manière à permettre, lorsque l'attache est poussée dans le tube dans le sens proximal-distal, une extraction dudit élément d'arrêt hors du tube, et le moyen de rétention est positionné de telle sorte qu'il assure la rétention de l'élément d'ancrage dans le tube lorsque l'élément d'arrêt est ainsi extrait hors du tube ; ledit moyen de rétention est tel qu'en deçà dudit seuil de force, il permet le recul de l'attache avec le tube, et que, une fois l'élément d'arrêt en appui contre la pièce prothétique et la pièce prothétique plaquée contre le support anatomique, il nécessite un surcroît de traction sur le tube pour libérer l'élément d'ancrage, assurant ainsi à cet élément d'ancrage une profondeur d'ancrage suffisante pour garantir que ladite pièce prothétique est bien plaquée contre ledit support anatomique.

De préférence, ladite conformation à contrainte minimum de l'attache correspond sensiblement à ladite conformation de démoulage de cette attache.

Avantageusement, dans ladite conformation à contrainte maximum, l'élément d'ancrage et l'élément d'arrêt sont disposés bout à bout, dans le prolongement l'un de l'autre, tandis que la tige de liaison s'étend parallèlement à la direction d'alignement de l'élément d'ancrage et de l'élément d'arrêt, entre les deux points de jonction avec respectivement l'élément d'ancrage et l'élément d'arrêt.

Grâce à l'invention, le geste opératoire requis pour la mise en place d'une attache au moins consiste simplement à faire pénétrer l'attache dans sa conformation à contrainte maximum, par exemple tubulaire, d'avant en arrière, dans la paroi tissulaire, puis à tirer la même attache d'arrière en avant, pour obtenir ou réobtenir une conformation non contrainte, ancrée dans la paroi tissulaire.

La conformation d'une attache selon l'invention permet par ailleurs de limiter la sollicitation angulaire cumulée lors du passage d'une conformation à l'autre, au niveau de l'articulation de la tige de liaison avec respectivement l'élément d'arrêt et l'élément d'ancrage.

L'invention sera mieux comprise par la description suivante par référence au dessin en annexe, dans lequel :
- la figure 1 représente un exemple de réalisation d'une attache conforme à l'invention, dans la conformation de démoulage,
- les figures 2 et 3 représentent un autre exemple de réalisation d'une attache conforme à l'invention, respectivement dans les conformations de démoulage et à contrainte maximum, sous forme tubulaire,
- la figure 3 bis représente une vue agrandie en coupe selon le plan IIIb de l'attache représentée en figure 3,
- la figure 4 schématise le mode d'insertion et de mise en place d'une attache conforme à l'invention dans un support anatomique,
- les figures 5 à 8 se rapportent à un autre mode d'exécution d'une attache selon l'invention, la Figure 5 représente, dans la conformation à contrainte maximum, une vue de face de l'attache, la figure 6, dans la conformation de démoulage, une vue de dessus de l'attache, et la figure 7, dans la même conformation, une vue de côté ; la figure 8 représente une vue agrandie d'un détail de la figure 7,
- les figures 9 et 10 représentent, respectivement dans la conformation de démoulage, et dans la conformation à contrainte maximum, tubulaire, un autre mode d'exécution d'une attache selon la présente invention,
- les figures 11 et 12 représentent, respectivement dans la conformation de démoulage, et dans la conformation à contrainte maximum, un autre mode d'exécution d'une attache selon la présente invention,
- la figure 13 représente un exemple de dispositif d'insertion transcutanée conforme à l'invention, pour la mise en place d'une attache conforme à l'invention dans un support anatomique,
- la figure 14 représente une variante d'exécution d'un dispositif d'insertion selon l'invention,
- les figures 15 à 18 représentent un autre exemple de dispositif d'insertion transcutanée conforme à l'invention, dans différentes phases de fonctionnement respectivement,
- la figure 19 représente un détail d'exécution du dispositif selon figures 15 à 18.

Les figures 20 à 23 représentent une variante du dispositif d'insertion représente aux figures 15 à 19, dans différentes phases de fonctionnement ; la figure 23b représente une vue en coupe selon la ligne A-A de la figure 23, d'un détail de ladite variante.

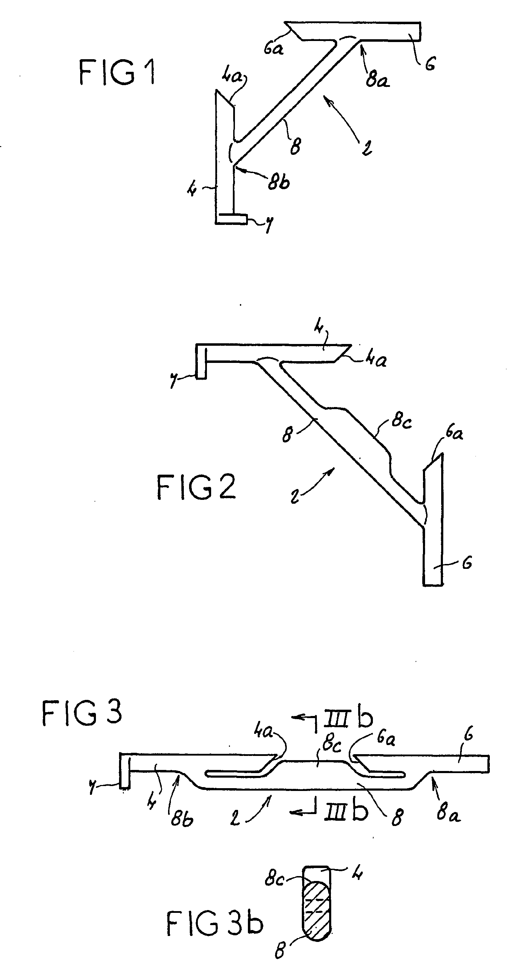

La figure 1 représente, dans la conformation de démoulage, une attache 2 comportant une élément d'ancrage 4 et un élément d'arrêt 6 relié par une tige de liaison 8.

Cette dernière est disposée entre un point intermédiaire de jonction 8b avec l'élément d'ancrage 4 et un point intermédiaire de jonction 8a avec l'élément d'arrêt 6.

Conformément à la figure 2, toujours dans la conformation de démoulage, la tige intermédiaire 8 présente un renflement 8c sensiblement en son centre. Ce renflement 8c consiste en une surépaisseur de matière, de longueur limitée par rapport à celle de la tige 8, orientée et disposée uniquement au dessus de l'âme de la tige 8, et à l'intérieur de l'attache 2, dans la conformation à contrainte maximum, tubulaire, représentée à la figure 3. Comme le montre cette dernière figure, dans la conformation tubulaire, le renflement 8c emplit ou occupe l'interstice laissé libre entre les deux extrémités respectivement proximale et distale de l'élément d'ancrage et de l'élément d'arrêt.

Les attaches 2 représentées aux figures 1 et 2 présentent une conformation à contrainte minimum, de retenue d'une pièce prothétique, dans laquelle les directions respectives de l'élément d'arrêt 6 et de l'élément d'ancrage 4 sont dans un même plan comprenant la tige de liaison 8, et forment entre elles, dans ledit plan, un angle au plus égal à 90 degrés. Dans les exemples de réalisations montrés aux figures 1 et 2, les attaches susceptibles d'être obtenues par moulage, sont montrées dans une conformation dite de moulage, identique à ou proche de la conformation à contrainte minimum précédemment décrite.

La figure 3 montre une attache 2 conforme à l'invention, dans la conformation à contrainte maximum, tubulaire, par insertion complète de ladite attache dans un tube (non représenté). Dans cette conformation, l'élément d'ancrage 4 et l'élément d'arrêt 6 sont disposés bout à bout dans le prolongement l'un de l'autre, et notamment de part et d'autre du renflement 8c. Dans cette conformation, la tige de liaison 8 s'étend parallèlement à la direction d'alignement de l'élément d'ancrage 4 et de l'élément d'arrêt 6, entre les deux points intermédiaires de jonction 8b, 8a avec respectivement l'élément d'ancrage 4 et l'élément d'arrêt 6.

Les extrémités de l'élément d'ancrage 4 et de l'élément d'arrêt 6, adjacentes dans la conformation contrainte tubulaire, comprennent respectivement deux pointes ou biseaux 4a, 6a.

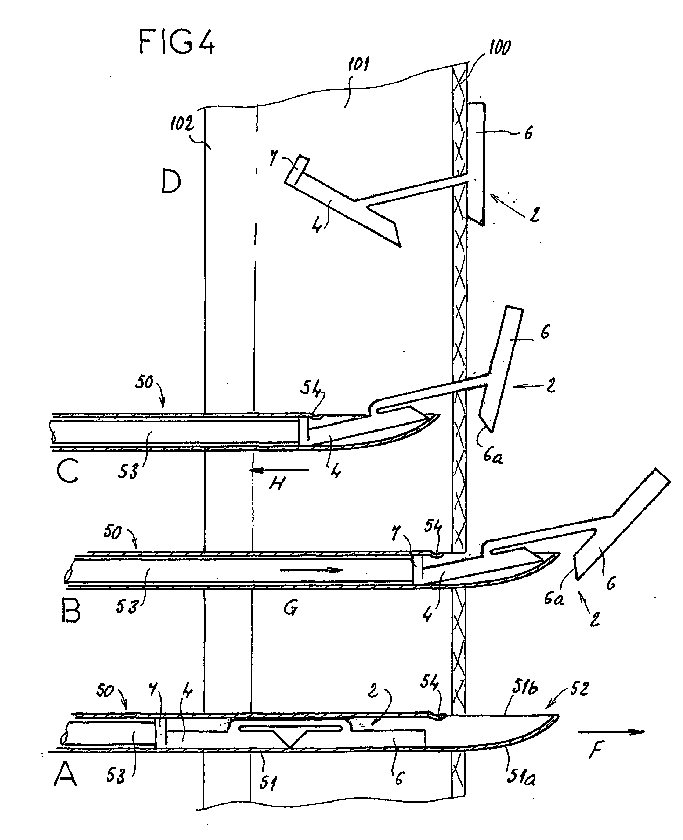

Dans la conformation à contrainte maximum, tubulaire, comme montré directement par la Figure 4 (vue du bas), ou indirectement par la Figure 3, les pointes ou biseaux 4a, 6a forment un angle aigu dirigé à l'opposé de la tige de liaison 8.

Dans l'exemple de réalisation représenté en conformation à contrainte maximum, tubulaire, à la figure 3, les deux extrémités adjacentes de l'élément d'ancrage 4 et de l'élément d'arrêt 6 demeurent distantes l'une de l'autre. L'élément d'ancrage 4 et l'élément d'arrêt 6 ont sensiblement la même longueur, tandis que chaque point intermédiaire de jonction 8a, 8b se situe sensiblement au milieu de l'élément d'ancrage distale 4 ou de l'élément d'arrêt 6.

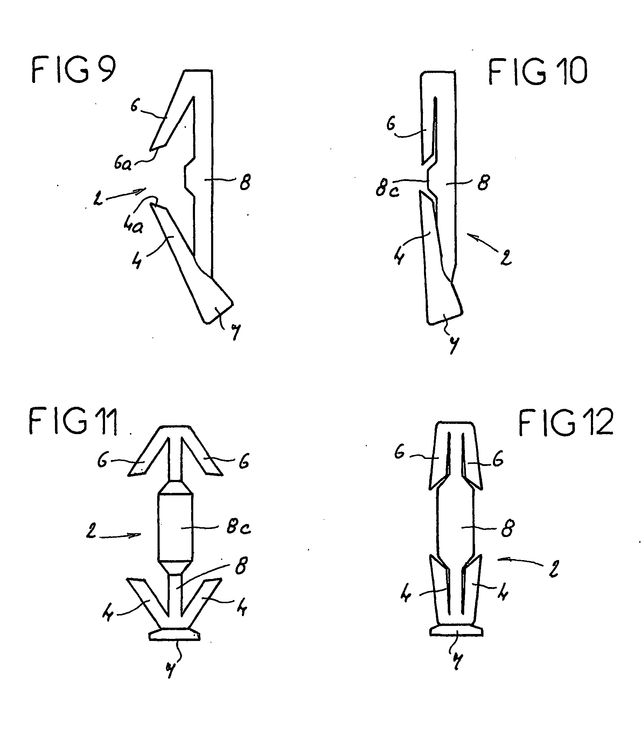

Comme montré en particulier par les figures 9 à 12, la jonction de l'élément d'ancrage 4 et/ou de l'élément d'arrêt 6, avec la tige de liaison 8, peut se situer à une extrémité ou en un point différent du milieu géométrique de ladite élément d'ancrage et/ou dudit élément d'arrêt.

Selon le mode de réalisation représenté à la figure 3, deux goussets sont ménagés sur la tige de liaison 8 du côté extérieur ou opposés aux deux points intermédiaires de jonction 8a et 8b respectivement avec l'élément d'ancrage 4 et l'élément d'arrêt 6.

L'élément d'ancrage 4 comporte par exemple une tête de poussée 7, disposée à l'extrémité proximale de ladite tige dans la conformation contrainte tubulaire.

Comme le montre la figure 4, une attache 2 selon l'invention est insérée au travers d'une paroi tissulaire ou musculaire 101, au moyen d'un dispositif d'insertion, désigné de manière générale par la référence 50, comprenant une aiguille 51 creuse. Cette aiguille ayant la forme d'une aiguille de Hubert comprend une extrémité distale 52 ouverte et acérée ; plus précisément la pointe de l'aiguille 51 est fermée par un embout concave 51 a et acéré, et comporte une ouverture latérale 51 b débouchante, en vis-à-vis de l'embout concave 51 a. La portion distale de l'aiguille 51, qui sert à perforer puis traverser la paroi tissulaire, continue et pleine en dehors de l'ouverture 51 b, en deçà de l'extrémité distale 52, permet de contenir au moins une attache 2 dans sa conformation à contrainte maximum, tubulaire. Comme montré par la figure 4, l'aiguille 51 comprend un moyen 54 de retenue (en traction et vers l'arrière) de l'attache 2. Ce moyen 54 consiste en un ou plusieurs pions obtenus par déformation de matière, arrêtant la tête de retenue prévue aussi à cet effet. Ce moyen de retenue est agencé en sorte de permettre un effacement relatif (entre lui-même et l'attache 2 ou sa tête 7), uniquement par traction et vers l'arrière de l'attache 2.

Ce ou ces pions peuvent être agencés par rapport à la tête 7, ou inversement, pour orienter angulairement l'agrafe par rapport à son axe, dans la conformation tubulaire.

Un mandrin de poussée 53 est contenu à l'intérieur de l'aiguille 50, et agencé pour coulisser librement à l'intérieur de cette dernière, en appui distal contre l'attache 2 dans sa conformation à contrainte maximum, tubulaire, et plus précisément sa tête de rétention 7. Le mandrin de poussée 53 comporte une extrémité proximale et extérieure, non représentée à la figure 4, pour sa commande directe en translation en poussée vers l'avant.

La figure 4 montre un exemple de mise en place d'une attache conforme à l'invention et plus particulièrement les phases de libération d'une attache 2 et de fixation d'une pièce prothétique 100, consistant pour l'essentiel en un tissu prothétique, dans le mode chirurgical mis en oeuvre dans le cadre de la présente invention.

Cette fixation se fait sur un support anatomique 101 consistant en une paroi tissulaire ou musculaire.

La mise en place d'une telle attache se fait par voie transtissulaire avec le dispositif d'insertion transcutanée 50, traversant la peau 102 du patient à partir de l'extérieur du corps.

L'attache 2 permet donc de fixer la pièce prothétique 100 tel que cela est montré à la phase D à la figure 4.

Lors d'une première phase A, décrite ci-après, l'aiguille 51 traverse la peau 102, puis la paroi tissulaire, ainsi que la pièce prothétique 100, de manière à pouvoir libérer l'élément d'arrêt 6 au-delà et en avant de ladite pièce prothétique 100, par l'intermédiaire de l'extrémité distale 52 ouverte et acérée, et ce à partir de la conformation tubulaire, à contrainte maximum montrée à la Figure 4A.

La méthode de pose des attaches 2 conforme à l'invention est explicitée par exemple à la figure 4 et peut se décomposer en quatre phases A, B, C et D.

Lors de la première phase A, le dispositif d'insertion permet d'introduire l'aiguille 51 en traversant la peau 102, la paroi tissulaire 101 et la pièce prothétique 100. L'aiguille 51 traverse la pièce prothétique 100 sur une distance suffisante pour permettre à l'extrémité distale 52 ouverte et acérée de libérer l'élément d'arrêt 6, par poussée avec le mandrin 53 selon la flèche G pendant la phase B, lequel s'écarte au moins en partie de sa conformation tubulaire contrainte après sa libération. Le mandrin de poussée 53 déplace l'attache 2, jusqu'à ce que l'extrémité biseautée 6a sorte de l'aiguille 51.

Conformément à la phase C de la figure 4, l'opérateur exerce une traction vers l'arrière sur le dispositif d'insertion 50 et, par conséquent, sur l'aiguille 51 selon la flèche H pour ramener l'élément d'arrêt 6 contre la pièce prothétique 100. Dès cet instant, l'attache 2, évolue vers sa conformation à contrainte minimum, relativement ouverte. L'élément d'arrêt 6 s'écarte donc sensiblement de sa position tubulaire contrainte, de manière à s'étendre sensiblement selon une direction orthogonale à celle de la tige de liaison 8.

Comme montré sous C à la figure 4, la forme biseautée 6a de l'extrémité de la tige d'arrêt 6, dirigée vers la pièce prothétique 100, permet par traction vers l'arrière de l'attache 2, (dont une partie demeure à l'intérieur de l'aiguille 51), de faire basculer la tige 6 dans une position à plat ou parallèle à la pièce 100 ou la paroi 101.

Ensuite, une traction supplémentaire vers l'arrière sur l'aiguille 51 permet d'effacer le moyen de retenue 54, par exemple par déformation ou par flexion de la tête de poussée 7 de l'attache 2, laquelle est alors libérée de l'aiguille 51. L'attache 2 se retrouve alors dans la conformation relativement ouverte, à contrainte minimum, schématisée à la phase D de la figure 4. Dans cette conformation, l'élément d'ancrage proximale 4 s'écarte également plus ou moins sensiblement de sa position dans la conformation de moulage de l'attache, pour s'ancrer dans la paroi 101.

Les pointes ou biseaux 6a et 4a respectivement de l'élément d'arrêt 6 et de l'élément d'ancrage 4 contribuent à une excellente insertion de ladite attache 2, dans tout support anatomique dans lequel vient se loger la tige proximale 4.

Il n'est pas nécessaire que l'élément d'ancrage 4 se déplie complètement pour prendre une direction angulaire perpendiculaire à celle de la tige de liaison 8, pour assurer un maintien ou une fixation du tissu prothétique 100 sur le support anatomique. Un faible écartement de l'élément d'ancrage 4 par rapport à sa position relative dans la conformation tubulaire contrainte de l'attache suffit.

L'existence du renflement 8c permet d'allonger la tige 8, sans pour autant l'affaiblir vis-à-vis des efforts longitudinaux qu'elle reçoit, aussi bien en poussée pour l'insertion et la mise en place de l'attache, dans la conformation tubulaire, à contrainte maximum, qu'en traction pour évoluer vers la conformation à contrainte minimum, de retenue de la pièce prothétique 100.

L'allongement de la tige 8 permet d'augmenter la profondeur de l'ancrage dans l'épaisseur de la paroi tissulaire 101 traversée, ce qui augmente l'épaisseur du matériau biologique, par exemple tissu, servant à la retenue de la prothèse grâce à l'attache mise en place.

La forme retenue pour les biseaux 4a et 6a permet, d'une part d'assurer la retenue de l'élément d'arrêt 6 dans la pièce prothétique précédemment traversée par l'attache 2, par basculement dudit barreau, forcé par traction arrière de l'attache, et d'autre part de favoriser l'ancrage de la tige 4, lorsque la tige 8 est sollicitée en traction, du fait du mouvement transversal de la pièce prothétique 100, et donc du mouvement transversal corrélatif de l'élément d'arrêt 6.

Comme le montre la figure 4, partie D, la tête 7 sert de moyen d'arrêt de l'ancrage dans la paroi tissulaire.

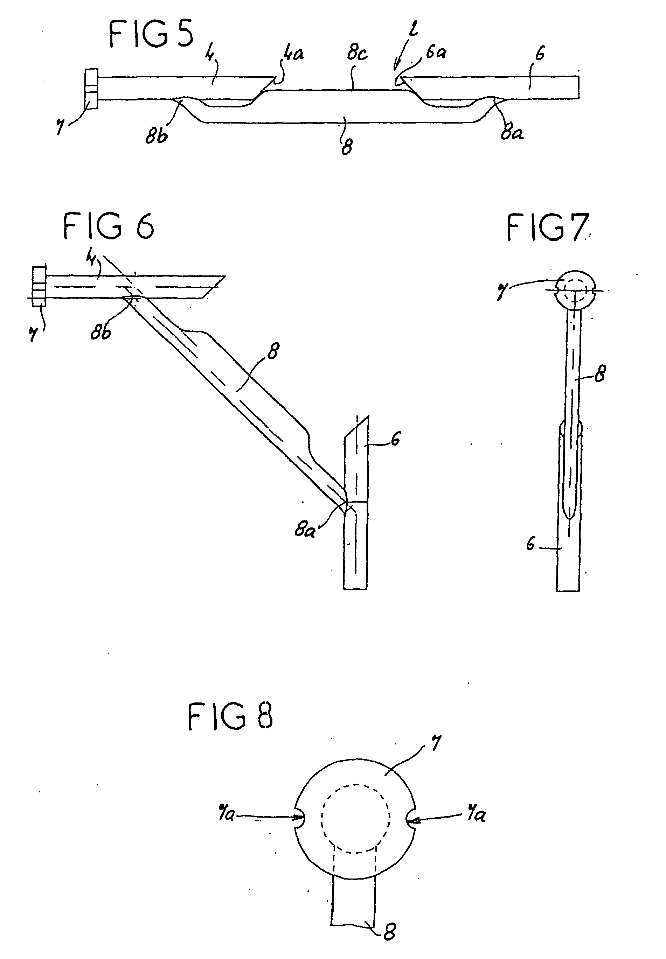

L'attache conforme aux figures 5 à 8 diffère de celle décrite par référence à la figure 1, par le fait que :
- dans la conformation à contrainte maximum, tubulaire, les biseaux 4a et 6a aboutent deux flancs obliques respectivement du renflement 8c,
- la tête 7 comporte deux encoches ou rainures périphériques, permettant le passage d'un gaz, par exemple de stérilisation, lorsque l'attache correspondante est dans la conformation contrainte et stockée à l'intérieur d'une aiguille 51.

L'attache 2, conforme aux figures 9 et 10 se caractérise, d'une part par une tige 4 d'ancrage et un élément d'arrêt 6 de section décroissante dans une direction s'éloignant de la tige de liaison 8, et d'autre part par un élément d'arrêt 6 articulé à son extrémité non libre sur la tige de liaison 8.

L'attache conforme aux figures 11 et 12 se caractérise par plusieurs barreaux d'arrêt 6 rayonnant et plusieurs tiges d'ancrage 4 rayonnant à partir des deux extrémités respectivement de la tige de liaison, le renflement 8c prenant la forme d'un moyeu.

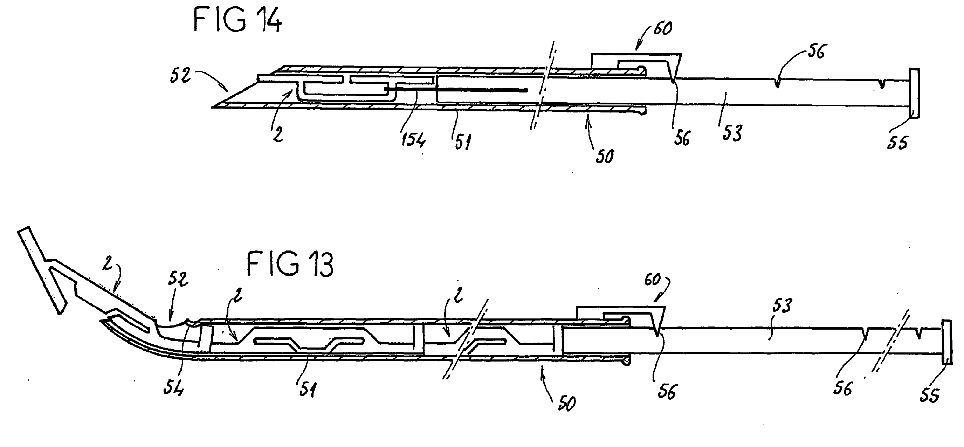

Les figures 13 et 14 représentent deux exemples de réalisation d'un dispositif d'insertion transtissulaire conforme à l'invention. Le dispositif d'insertion transcutanée 50 représenté à la figure 13 comprend l'aiguille 51 présentant l'extrémité distale ouverte et acérée 52 d'une part, et une extrémité dans laquelle est introduit le mandrin de poussée 53, à l'extrémité extérieure et proximale duquel est disposé un organe de commande 55.

La portion distale de pénétration de l'aiguille 51 comprend une charge ou une pluralité d'attaches 2 stockées bout à bout, chacune dans leur conformation tubulaire à contrainte maximum, à l'intérieur de l'aiguille creuse 51.

Le dispositif d'insertion transcutanée 50 comporte également un moyen de repérage gradué 60 de l'avancement du mandrin de poussée 53 à l'intérieur de l'aiguille 51, permettant d'indiquer à l'opérateur le nombre d'attaches 2 utilisées. Ce moyen 60 permet de compter le nombre d'attaches 2 libérées lors de la fixation de la pièce prothétique 100 sur le support anatomique, en l'occurrence la paroi tissulaire 101.

Le moyen de retenue 54 est dimensionné de façon à retenir la tête de poussée 7 d'une manière suffisante à positionner de façon optimale l'élément d'arrêt 6 contre la pièce prothétique 100.

L'exemple de réalisation du dispositif d'insertion représenté à la figure 14 ne comporte qu'une seule attache 2 dans l'aiguille 51. A la différence du moyen de retenue référencé 54 à la figure 13, celui-ci référencé 154 à la figure 14, est réalisé avec une tige ou un fil reliant ladite attache 2 au mandrin de poussée 53.

Les dispositifs d'insertion 50 des figures 13 et 14 sont à usage unique, avec une seule attache 2 conforme à l'invention dans le mode d'exécution selon figure 14 et avec une charge de plusieurs attaches dans le mode d'exécution selon figure 13.

Les figures 15 à 19 représentent un autre exemple de réalisation d'un dispositif d'insertion transtissulaire conforme à l'invention. Le dispositif d'insertion comprend un manche 70, à une extrémité distale 71 duquel l'aiguille 51 est montée coaxialement. Le montage de l'aiguille 51 sur le manche 70 est obtenu à l'aide d'une pièce de montage 72 solidaire de l'aiguille 51, et engagée au moins en partie dans ledit manche de commande 70. Cette pièce de montage 72 est solidarisée avec l'extrémité distale 71 dudit manche 70 par tout moyen connu, et notamment par vissage ou collage.

L'aiguille 51 est traversée coaxialement et librement par le mandrin de poussée 53. Ce dernier est en appui du côté distal contre une attache 2 et est disposé du côté proximal librement à l'intérieur d'un coulisseau 74 monté de manière libre et en translation, coaxialement dans le manche 70. Le mandrin est solidaire à son extrémité proximale d'un noyau ou contre-butée 90, coaxial à l'aiguille 51 et au mandrin 53.

Le coulisseau 74 comprend selon son axe une série de butées axiales 74a, 74b et 74c, radialement élastiques vers l'extérieur, en nombre identique moins un à celui des attaches 2 disposées dans la portion distale de pénétration de l'aiguille 51. Les butées axiales 74a, 74b et 74c sont espacées selon l'axe du coulisseau 74 et séparées par une même distance égale à la longueur d'une attache 2 dans sa conformation contrainte tubulaire, laquelle détermine la course élémentaire d'avancée du mandrin de poussée 53.

Un organe de poussée 80 émerge pour une partie 80b du manche 70, et est monté mobile en translation coaxialement dans ce dernier, en appui du côté distal contre l'extrémité du coulisseau 74 opposée à l'aiguille 51. L'organe 80 comprend à l'intérieur du manche 70 une face distale 80a, opposée à la partie émergente 80b, d'appui contre le coulisseau 74 et contre la contre-butée 90, avant la première utilisation du dispositif d'insertion.

Ce dispositif comprend un moyen de rappel 82 du coulisseau 74 dans une position de repos, dans laquelle l'extrémité proximale 74d du coulisseau 74 et la face distale 80a de l'organe 80 de préhension sont en appui l'une contre l'autre. Le moyen de rappel 82 est par exemple un ressort monté dans le manche 70, et ce entre l'extrémité distale du coulisseau 74, par l'intermédiaire d'une rondelle 91, et l'extrémité distale 71 du manche 70.

Un moyen anti-recul 84 est prévu entre l'extrémité distale 71 du manche 70 et le mandrin de poussée 53. Le moyen anti-recul 84 permet le mouvement dudit mandrin de poussée 53 en avant dudit manche 70 et bloque son mouvement en arrière, toujours par rapport audit manche. Le mandrin de poussée 53 se trouve donc bloqué en translation dans chacune de ses positions d'avancée élémentaire, correspondant chacune à l'extraction complète d'une attache 2 de l'aiguille 51. Chacune de ces positions élémentaires est matérialisée par chacune de la face distale 80a et des butées axiales 74a, 74b, 74c dans la position de rappel du coulisseau 74.

Chacune des butées 74a à 74c est agencée pour échapper à la contre-butée 80, lorsque le mandrin de poussée 53 est arrêté en translation par le moyen anti-recul 84, et lorsque le coulisseau 74 est rappelé dans sa position de rappel ou repos. A cette fin, chaque butée 74a à 74c consiste en une ou plusieurs dents élastiques radialement, comportant un talon interne 95 profilé pour échapper à la contre-butée 90 lorsque le coulisseau 74 recule sous l'action du ressort 82, et pour résister à la contre-butée 90 lorsque le coulisseau 74 avance, et que la face frontale et distale de chaque dent élastique appuie contre la contre-butée 90.

Lorsque l'opérateur souhaite extraire une attache 2 de l'aiguille 51 avec le dispositif d'insertion transcutanée précédemment décrit, il part d'une position représentée à la figure 15. Il appuie ensuite sur l'organe de poussée 80 pour comprimer le moyen de rappel 82 et déplacer le mandrin de poussée 53 par translation de manière à expulser l'élément d'arrêt 6 hors de l'aiguille 51. La course de l'organe 80 permet de déplacer le mandrin de poussée 53, de manière à faire venir sensiblement en contact la tête de poussée 7 et le moyen de retenue 54 pour la première attache 2 destinée à être extraite de l'aiguille 51. On se retrouve alors dans la disposition représentée à la figure16.

L'opérateur peut alors exercer une traction sur l'aiguille 51 par l'intermédiaire du manche 70 pour positionner l'attache 2 contre la pièce prothétique 100 avec l'élément d'arrêt 6, donc contre la paroi tissulaire 101. En exerçant une traction vers l'arrière, celle-ci permet d'effacer la tête de poussée 7 par rapport au moyen de retenue 54.

Lorsque l'opérateur relâche ensuite l'organe 80, ce dernier revient dans sa position de repos par l'action du moyen de rappel 82. Le déplacement du coulisseau 74 à l'intérieur du manche 70 se fait sans mouvement relatif entre le mandrin de poussée 53 et le manche 70, et ce grâce au moyen anti-recul 84.

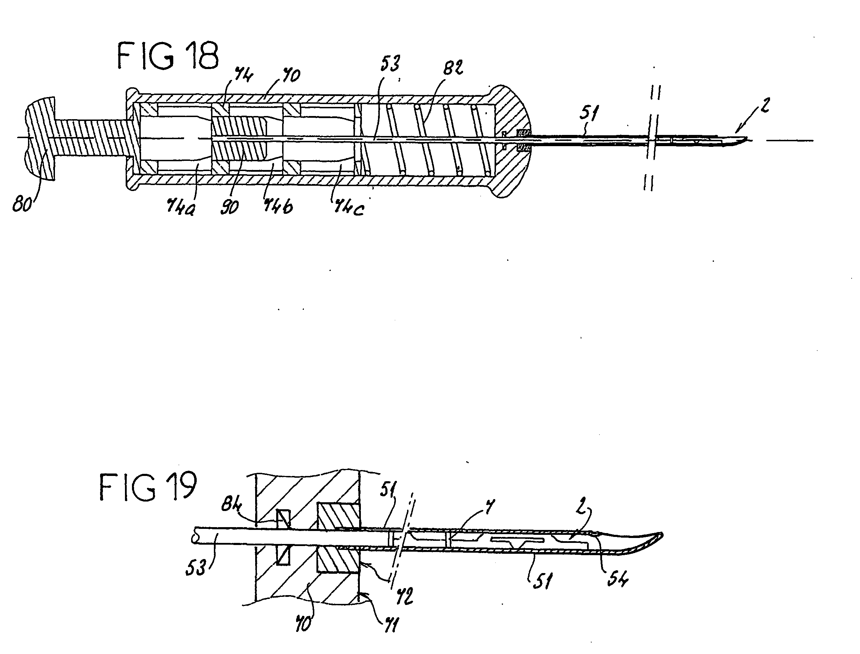

La contre-butée 90 vient alors se loger en avant de la première butée 74a prévue à l'intérieur du coulisseau 74, comme montré successivement par les figures 17 et 18. C'est dans cette nouvelle position que la contre-butée 90 vient se loger du côté de la seconde butée 74b, et prendre appui contre la première butée 74a, lors d'une nouvelle avancée élémentaire de l'organe de poussée 80.

Le dispositif d'insertion se retrouve alors dans une position d'attente représentée à la figure 18.

Selon le mode opératoire de pose décrit à la figure 4, lorsque l'attache 2 est entièrement extraite de l'aiguille 51, le dispositif d'insertion représenté aux figures 15 à 18 se trouve alors dans une disposition dans laquelle il est possible d'extraire une seconde attache 2. En effet, lors d'une poussée sur l'organe de préhension 80, le coulisseau 74 par l'intermédiaire de la première butée axiale 74a déplace par translation le mandrin de poussée 53 dans une deuxième position élémentaire située en avant et au-delà de la position de la deuxième butée axiale 74b lorsque le coulisseau 74 est au repos. Ainsi, lorsque l'élément d'arrêt 6 de la seconde attache 2 est expulsé de l'aiguille 51, et que la tête de poussée 7 de la seconde attache 2 vient en contact avec le moyen de retenue 54, l'opérateur relâche l'organe 80, lequel sous l'effet du moyen de rappel 82 dispose à nouveau le coulisseau 74 dans sa position de repos. La contre-butée 90 vient alors se loger en avant de la seconde butée axiale 74b. La libération complète de la deuxième attache 2 peut ainsi être obtenue, et une attache supplémentaire, en l'occurrence une troisième attache 2 pourra ensuite être extraite de l'aiguille 51, et ainsi de suite Le dispositif d'insertion conforme à l'invention et représenté aux figures 15 à 19, ne se limite pas à un exemple permettant la pose de quatre attaches 2. Bien au contraire, l'aiguille 51 correspondante peut comprendre des charges d'un nombre variable d'attaches. La taille du dispositif d'insertion, ainsi que le diamètre de l'aiguille correspondante peuvent également varier sans sortir du cadre de la présente invention. A titre d'exemple, l'aiguille 51 peut présenter un diamètre intérieur de 1,6 mm.

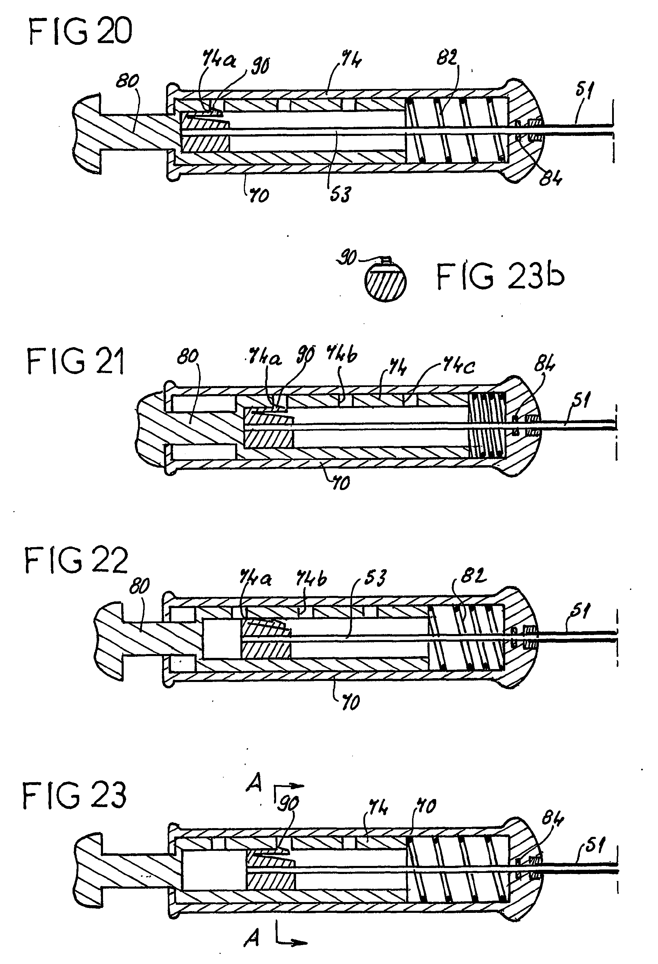

Le dispositif représenté aux figures 21 à 23 et 23b diffère du dispositif précédemment décrit, par les dispositions suivantes :
- au lieu de constituer une pièce distincte du coulisseau 74, l'organe de poussée 80 est solidaire de, ou forme une seule pièce monobloc avec le coulisseau
- pour échapper à la contre-butée 90, lorsque le coulisseau 74 recule vers sa position de repos d'une part, chaque butée axiale 74a, 74b, 74c, appartenant au coulisseau 74 est constituée par un cran d'arrêt constitué par un perçage traversant (fente circulaire par exemple) la paroi du coulisseau 74 creux, et d'autre part la contre-butée 90 est constituée par une ou plusieurs dents élastiques radialement vers l'extérieur, une telle dent ayant un méplat de butée contre le cran d'arrêt précité, dans le sens de la poussée de l'organe 80, et un biseau pour glisser contre la face interne du coulisseau 74, dans le sens opposé.

## Revendications

1. Attache (2) de fixation prothétique, ayant une structure monolithique, susceptible d'être obtenue par moulage dans un matériau plastique, par exemple biorésorbable, et composée d'au moins un élément (4) d'ancrage dans un support anatomique, d'au moins un élément (6) d'arrêt par rapport à une pièce prothétique (100) plane à fixer, et d'une tige de liaison (8) disposée entre une zone (8b) de jonction avec l'élément d'ancrage (4) et une zone (8a) de jonction avec l'élément d'arrêt (21) ;
la tige de liaison (8) étant rectiligne, attache **caractérisée en ce que** les zones (8a, 8b) de jonction de l'élément d'ancrage (4) et de l'élément d'arrêt (6) avec cette tige de liaison (8) permettent à l'attache de prendre au moins trois conformations distinctes, à savoir :
- une conformation de démoulage, non contrainte, dans laquelle les directions respectives de l'élément d'arrêt (6) et de l'élément d'ancrage (4) forment entre elles, dans un même plan comprenant la tige de liaison (8), un angle intérieur au moins égal à 90° ;
- une conformation à contrainte maximum, ramassée sur elle-même, par exemple tubulaire par insertion complète de ladite attache dans un tube (51), dans laquelle l'élément d'ancrage (4) et l'élément d'arrêt (6) sont repliés vers la tige de liaison (8) et l'un vers l'autre, quasiment bout à bout et dans le prolongement l'un de l'autre; et
- une conformation à contrainte minimum, dans laquelle les directions respectives de l'élément d'arrêt (6) et de l'élément d'ancrage (4) forment entre elles, dans un même plan, un angle au plus égal à 90°.

2. Attache selon la revendication 1, **caractérisé en ce que** la conformation à contrainte minimum correspond sensiblement à la conformation de démoulage.

3. Attache selon la revendication 1, **caractérisé en ce que**, dans ladite conformation à contrainte maximum, l'élément d'ancrage (4) et l'élément d'arrêt (6) sont disposés bout à bout, dans le prolongement l'un de l'autre, tandis que la tige de liaison (8) s'étend parallèlement à la 1 direction d'alignement de l'élément d'ancrage et de l'élément d'arrêt, entre les deux points de jonction avec respectivement l'élément d'ancrage et l'élément d'arrêt.

4. Attache selon la revendication 1, **caractérisée en ce que** les extrémités de l'élément d'ancrage (4) et de l'élément d'arrêt (6), adjacentes dans la conformation à contrainte maximum, comprennent respectivement deux pointes ou biseaux (6a, 4a).

5. Attache selon la revendication 4, **caractérisé en ce que** dans la conformation à contrainte maximum, les pointes ou biseaux (5a, 4a) forment un angle intérieur.

6. Attache selon la revendication 1, **caractérisée en ce que** deux goussets sont ménagés sur la tige de liaison, du côté extérieur des deux points intermédiaires de jonction, respectivement avec l'élément d'ancrage (4) et l'élément d'arrêt (6).

7. Attache selon la revendication 1, **caractérisée en ce que**, dans la conformation à contrainte maximum, les deux extrémités adjacentes de l'élément d'ancrage (4) et de l'élément d'arrêt (6) demeurent distantes l'une de l'autre.

8. Attache selon la revendication 1, **caractérisée en ce que** l'élément d'ancrage comporte une tête de rétention (7), disposée à l'extrémité proximale de ladite élément d'ancrage (4), dans la conformation contrainte maximum tubulaire.

9. Attache selon la revendication 1, **caractérisée en ce que** l'élément d'ancrage (4) et l'élément d'arrêt (6) ont sensiblement la même longueur, tandis que chaque point de jonction (8a, 8b) avec la tige de liaison (8) se situe sensiblement au milieu de l'élément d'ancrage (4) ou de l'élément d'arrêt (6).

10. Attache selon la revendication 1, **caractérisée en ce que** la tige de liaison (8) comprend un renflement (8c) séparant l'élément d'ancrage (4) et l'élément d'arrêt (6), dans la conformation à contrainte maximum de ladite attache.

11. Dispositif de mise en place d'une attache selon l'une quelconque des revendications 1 à 10, **caractérisé en ce qu'**il comprend le tube (51) et l'attache (2) précités, celle-ci étant insérée dans ce tube (51) avec ledit élément d'arrêt (6) tourné vers l'extrémité distale de ce tube (51) et ledit élément d'ancrage (4) tourné vers l'extrémité proximale de ce tube (51) ; ce tube (51) comporte une extrémité distale (52) acérée, présentant une ouverture latérale, et est pourvu d'un moyen (54) de rétention de l'élément d'ancrage (4) en deçà d'un seuil de force ; l'ouverture latérale est dimensionnée de manière à permettre, lorsque l'attache (2) est poussée dans le tube (51) dans le sens proximal-distal, une extraction dudit élément d'arrêt (6) hors du tube, et le moyen (54) de rétention est positionné de telle sorte qu'il assure la rétention de l'élément d'ancrage (4) dans le tube lorsque l'élément d'arrêt (6) est ainsi extrait hors du tube ; ledit moyen (54) de rétention est tel qu'en deçà dudit seuil de force, il permet le recul de l'attache (2) avec le tube (51), et que, une fois l'élément d'arrêt (6) en appui contre la pièce prothétique (100) et la pièce prothétique (100) plaquée contre le support anatomique (101), il nécessite un surcroît de traction sur le tube (51) pour libérer l'élément d'ancrage (4), assurant ainsi à cet élément d'ancrage (4) une profondeur d'ancrage suffisante pour garantir que ladite pièce prothétique (100) est bien plaquée contre ledit support anatomique (101).

12. Dispositif selon la revendication 11, **caractérisé en ce que** le tube (51) comporte une extrémité distale (52) ouverte est acérée et **en ce que** la portion distale de pénétration de ce tube (51) est continue et pleine en deçà de cette extrémité distale, en sorte de pouvoir contenir complètement au moins une dite attache (2) dans sa conformation tubulaire contrainte.

13. Dispositif selon la revendication 11 ou 12, **caractérisé en ce que** la portion distale de pénétration de l'aiguille (51) comprend une charge ou pluralité d'attaches (2) disposées bout à bout, chacune dans leur conformation tubulaire contrainte.

14. Dispositif selon l'une des revendications 11 à 13, **caractérisé en ce que**, outre l'aiguille, il comprend un mandrin de poussée (53) de l'extrémité proximale d'une attache disposée dans sa conformation tubulaire contrainte dans la portion distale de pénétration de ladite aiguille, ledit mandrin de poussée étant adapté pour pénétrer et se déplacer librement à l'intérieur de ladite aiguille, et comportant une extrémité proximale et extérieure pour sa commande, directe ou indirecte.

15. Dispositif selon la revendication 14, **caractérisé en ce** l'extrémité extérieure et proximale du mandrin de poussée (53) comprend un organe de commande.

16. Dispositif selon l'une des revendications 11 à 15, **caractérisé en ce que** la portion distale de pénétration comprend une charge ou pluralité d'attaches (2) disposées bout à bout, chacune dans leur disposition tubulaire contrainte.

17. Dispositif selon l'une des revendications 14 à 16, **caractérisé en ce qu'**il comporte un moyen de repérage gradué de l'avancement du mandrin de poussée (53) à l'intérieur de l'aiguille (51).

18. Dispositif selon l'une des revendications 14 à 17, **caractérisé en ce qu'**il comprend :
- un manche (70) sur lequel l'aiguille (51) est montée coaxialement, à une extrémité distale dudit manche,
- un coulisseau (74) monté de manière libre en translation, coaxialement à l'intérieur dudit manche (70), ledit coulisseau comprenant une série de butées axiales (74a, 74b, 74c), en nombre identique moins un à celui des attaches (2) disposées dans la portion distale de pénétration de l'aiguille (51), distribuées et séparées selon l'axe du manche par une même distance correspondant à la course élémentaire d'avance du mandrin de poussée (53), défini ci-après,
- un organe de poussée (80) émergeant dudit manche, monté mobile en translation à l'intérieur dudit manche, de l'autre côté du coulisseau par rapport à l'aiguille (51),
- un moyen de rappel du coulisseau (82) dans une position de repos ou rappel, dans laquelle l'extrémité proximale du coulisseau (74) et l'extrémité distale de l'organe de préhension (80) sont en butée l'une contre l'autre,
- le mandrin de poussée (53) disposé coaxialement, du côté distal à l'intérieur de l'aiguille, solidaire du côté proximal d'une contre-butée (90).
- un moyen anti-recul (84) entre le manche (70) et le mandrin de poussée (53), permettant le mouvement en translation dudit mandrin en avant dudit manche (70), et bloquant son mouvement en arrière, toujours par rapport audit manche,
- chacune des butées (74a, 74b, 74c) axiales du coulisseau étant agencée pour échapper à la dite contre-butée (90), lorsque, le mandrin étant bloqué en translation par le moyen anti-recul, le coulisseau est rappelé en arrière dans sa position de repos.

19. Dispositif selon la revendication 18, **caractérisé en ce que** l'organe de poussée (80) est distinct du coulisseau (74), ou constitue une seule pièce monobloc avec ledit coulisseau (74).

20. Dispositif selon la revendication 18, **caractérisé en ce que**, pour échapper à la contre butée (90), chaque butée axiale (74a, 74b, 74c) est constituée par une ou plusieurs dents élastiques radialement vers l'extérieur, ou chaque butée axiale (74a, 74b, 74c) est constituée par un cran d'arrêt, et la contre-buté est constituée par au moins une dent élastique radialement vers l'intérieur.

## Claims

1. A fastener (2) for fixing a prosthesis, having a single-piece structure that can be obtained by molding of a plastics material, for example a bioabsorbable material, and composed of at least one element (4) for anchoring in an anatomical support, at least one element (6) for immobilization relative to a planar prosthetic component (100) to be fixed, and a linking rod (8) arranged between a zone (8b) of connection to the anchoring element (4) and a zone (8a) of connection to the immobilizing element (21); the linking rod (8) being rectilinear, said fastener being **characterized in that** the zones (8a, 8b) of connection of the anchoring element (4) and of the immobilizing element (6) to this linking rod (8) allow the fastener to adopt at least three distinct configurations, namely:
- a non-stressed mold-release configuration in which the respective directions of the immobilizing element (6) and of the anchoring element (4) form between them, in the same plane comprising the linking rod (8), an internal angle of at least 90°;
- a configuration of maximum stress, doubled back on itself, for example tubular, by complete insertion of said fastener into a tube (51), in which configuration the anchoring element (4) and the immobilizing element (6) are folded toward the linking rod (8) and toward one another, as it were end-to-end and in a continuation of one another; and
- a configuration of minimum stress in which the respective directions of the immobilizing element (6) and of the anchoring element (4) form between them, for example in the same plane, an angle not greater than 90°.

2. The fastener as claimed in claim 1, **characterized in that** the configuration of minimum stress corresponds substantially to the mold-release configuration.

3. The fastener as claimed in claim 1, **characterized in that**, in said configuration of maximum stress, the anchoring element (4) and the immobilizing element (6) are arranged end-to-end, in a continuation of one another, while the linking rod (8) extends parallel to the direction of alignment of the anchoring element and of the immobilizing element, between the two points of connection to, respectively, the anchoring element and the immobilizing element.

4. The fastener as claimed in claim 1, **characterized in that** those ends of the anchoring element (4) and of the immobilizing element (6) which are adjacent in the configuration of maximum stress comprise two points or bevels (6a, 4a), respectively.

5. The fastener as claimed in claim 4, **characterized in that**, in the configuration of maximum stress, the points or bevels (5a, 4a) form an internal angle.

6. The fastener as claimed in claim 1, **characterized in that** two gussets are formed on the linking rod, to the outside of the two intermediate points of connection to the anchoring element (4) and to immobilizing element (6), respectively.

7. The fastener as claimed in claim 1, **characterized in that**, in the configuration of maximum stress, the two adjacent ends of the anchoring element (4) and of the immobilizing element (6) remain at a distance from one another.

8. The fastener as claimed in claim 1, **characterized in that** the anchoring element comprises a retention head (7) arranged at the proximal end of said anchoring element (4) in the tubular maximum stressed configuration.

9. The fastener as claimed in claim 1, **characterized in that** the anchoring element (4) and the immobilizing element (6) have substantially the same length, while each point of connection (8a, 8b) to the linking rod (8) is situated substantially at the center of the anchoring element (4) or immobilizing element (6).

10. The fastener as claimed in claim 1, **characterized in that** the linking rod (8) comprises a bulge (8c) separating the anchoring element (4) and the immobilizing element (6), in the configuration of maximum stress of said fastener.

11. A device for delivering a fastener as claimed in any one of claims 1 through 10, **characterized in that** it comprises the tube (51) and the fastener (2) mentioned above, the fastener (2) being inserted into this tube (51) with said immobilizing element (6) directed toward the distal end of this tube (51) and said anchoring element (4) directed toward the proximal end of this tube (51); this tube (51) comprises a pointed distal end (52) having a lateral opening, and it is provided with a means (54) for retention of the anchoring element (4) below a threshold force; the lateral opening is dimensioned in such a way as to allow extraction of said immobilizing element (6) from the tube when the fastener (2) is pushed into the tube (51) in the proximal to distal direction, and the retention means (54) is positioned in such a way that it ensures retention of the anchoring element (4) in the tube when the immobilizing element (6) is thus extracted from the tube; said retention means (54) is such that, below said threshold force, it permits the retreat of the fastener (2) with the tube (51) and that, once the immobilizing element (6) is bearing against the prosthetic component (100) and the prosthetic component (100) is pressed against the anatomical support (101), it requires an increased traction on the tube (51) in order to release the anchoring element (4), thereby ensuring that this anchoring element (4) has a depth of anchoring sufficient to guarantee that said prosthetic component (100) is pressed sufficiently against said anatomical support (101).

12. The device as claimed in claim 11, **characterized in that** the tube (51) comprises an open is pointed distal end (52) and **in that** the distal penetrating portion of this tube (51) is continuous and solid on this side of this distal end, so as to be able to completely contain at least one said fastener (2) in its stressed tubular configuration.

13. The device as claimed in claim 11 or 12, **characterized in that** the distal penetrating portion of the needle (51) comprises a charge or plurality of fasteners (2) arranged end-to-end, each in their stressed tubular configuration.

14. The device as claimed in one of claims 11 through 13, **characterized in that**, in addition to the needle, it comprises a pusher mandrel (53) for pushing the proximal end of a fastener arranged in its stressed tubular configuration in the distal penetrating portion of said needle, said pusher mandrel being designed to penetrate into and move freely inside said needle, and comprising a proximal and external end for its direct or indirect control.

15. The device as claimed in claim 14, **characterized in that** the external and proximal end of the pusher mandrel (53) comprises a control member.

16. The device as claimed in one of claims 11 through 15, **characterized in that** the distal penetrating portion comprises a charge or plurality of fasteners (2) arranged end-to-end, each in their stressed tubular configuration.

17. The device as claimed in one of claims 14 through 16, **characterized in that** it comprises a graduated means of registering the advance of the pusher mandrel (53) inside the needle (51).

18. The device as claimed in one of claims 14 through 17, **characterized in that** it comprises:
- a handle (70) on which the needle (51) is mounted coaxially, at a distal end of said handle,
- a slide (74) mounted freely in translation, coaxially inside said handle (70), said slide comprising a series of axial stops (74a, 74b, 74c) which are in identical number, less one, to that of the fasteners (2) arranged in the distal penetrating portion of the needle (51) and which are distributed and separated along the axis of the handle by a same distance corresponding to the basic forward travel of the pusher mandrel (53), as defined below,
- a pusher member (80) emerging from said handle and mounted so as to be movable in translation inside said handle, on the other side of the slide in relation to the needle (51),
- a means (82) of returning the slide to a rest or return position in which the proximal end of the slide (74) and the distal end of the gripping member (80) are in abutment against one another,
- the pusher mandrel (53) arranged coaxially, at the distal end inside the needle, and integral at the proximal end with a counter-abutment (90).
- a non-return means (84) between the handle (70) and the pusher mandrel (53), allowing said mandrel to move in translation forward of said handle (70) and blocking its rearward movement, again in relation to said handle,
- each of the axial stops (74a, 74b, 74c) of the slide being arranged to escape from said counter-abutment (90) when, with the mandrel blocked in translation by the non-return means, the slide is drawn back to its rest position.

19. The device as claimed in claim 18, **characterized in that** the pusher member (80) is separate from the slide (74) or forms a single piece together with said slide (74).

20. The device as claimed in claim 18, **characterized in that**, in order to escape from the counter-abutment (90), each axial stop (74a, 74b, 74c) is made up of one or more radially outwardly elastic teeth, or each axial stop (74a, 74b, 74c) is formed by a catch and the counter-abutment is formed by at least one radially inwardly elastic tooth.

## Patentansprüche

1. Prothetische Befestigungsklammer (2), die eine einstückige Struktur aufweist, die durch Formung aus einem beispielsweise bioresorbierbaren Kunststoff gefertigt werden kann, und die aufgebaut ist aus zumindest einem Verankerungselement (4) zur Verankerung in einem anatomischen Träger, aus zumindest einem Festlegeelement (6) zum Festlegen bezüglich eines zu befestigenden ebenen prothetischen Stücks (100), und aus einem Verbindungsschaft (8), der zwischen einer Verbindungszone (8b) mit dem Verankerungselement (4) und einer Verbindungszone (8a) mit dem Festlegeelement (21) angeordnet ist;
wobei der Verbindungsschaft (8) gerade ist,
wobei die Klammer **dadurch gekennzeichnet ist, dass** die Verbindungszonen (8a, 8b) des Verankerungselements (4) und des Festlegeelements (6) mit diesem Verbindungsschaft (8) es ermöglichen, dass die Klammer zumindest drei verschiedene Formzustände einnimmt, und zwar:
- einen nicht gespannten Abformungsformzustand, in dem die jeweiligen Richtungen des Festlegeelements (6) und des Verankerungselements (4) in einer selben Ebene, die den Verbindungsschaft (8) enthält, untereinander einen Innenwinkel von zumindest gleich 90° bilden;
- einen Formzustand mit maximaler Spannung, der auf sich selbst zusammengelegt, beispielsweise durch vollständiges Einsetzen der Klammer in ein Rohr (51) rohrförmig ist, in dem das Verankerungselement (4) und das Festlegeelement (6) zu dem Verbindungsschaft (8) hin und zueinander hin, gewissermaßen Ende an Ende und in Verlängerung voneinander gefaltet sind; und
- einen Formzustand mit minimaler Spannung, in der die jeweiligen Richtungen des Festlegeelements (6) und des Verankerungselements (4) in einer selben Ebene untereinander einen Winkel von höchstens gleich 90° bilden.

2. Klammer nach Anspruch 1, **dadurch gekennzeichnet, dass** der Formzustand mit minimaler Spannung etwa dem Abformungsformzustand entspricht.

3. Klammer nach Anspruch 1, **dadurch gekennzeichnet, dass** das Verankerungselement (4) und das Festlegeelement (6) in dem Formzustand mit maximaler Spannung Ende an Ende in Verlängerung zueinander angeordnet sind, während sich der Verbindungsschaft (8) parallel zu der Richtung der Ausrichtung des Verankerungselements und des Festlegeelements zwischen den beiden Verbindungsstellen mit dem Verankerungselement bzw. dem Festlegeelement erstreckt.

4. Klammer nach Anspruch 1, **dadurch gekennzeichnet, dass** die benachbarten Enden des Verankerungselements (4) und des Festlegeelements (6) in dem Formzustand mit maximaler Spannung zwei Spitzen oder abgeschrägte Kanten (6a, 4a) aufweisen.

5. Klammer nach Anspruch 4, **dadurch gekennzeichnet, dass** die Spitzen oder abgeschrägten Kanten (5a, 4a) in dem Formzustand mit maximaler Spannung einen Innenwinkel bilden.

6. Klammer nach Anspruch 1, **dadurch gekennzeichnet, dass** zwei Knotenstücke an dem Verbindungsschaft auf der Außenseite der beiden dazwischen liegenden Verbindungsstellen mit dem Verankerungselement (4) bzw. dem Festlegeelement (6) ausgebildet sind.

7. Klammer nach Anspruch 1, **dadurch gekennzeichnet, dass** die beiden benachbarten Enden des Verankerungselements (4) und des Festlegeelements (6) in dem Formzustand mit maximaler Spannung voneinander beabstandet bleiben.

8. Klammer nach Anspruch 1, **dadurch gekennzeichnet**, das das Verankerungselement einen Rückhaltekopf (7) aufweist, der in dem maximal gespannten rohrförmigen Zustand an dem proximalen Ende des Verankerungselements (4) angeordnet ist.

9. Klammer nach Anspruch 1, **dadurch gekennzeichnet, dass** das Verankerungselement (4) und das Festlegeelement (6) etwa die gleiche Länge aufweisen, während jede Verbindungsstelle (8a, 8b) mit dem Verbindungsschaft (8) sich etwa in der Mitte des Verankerungselements (4) bzw. des Festlegeelements (6) befindet.

10. Klammer nach Anspruch 1, **dadurch gekennzeichnet, dass** der Verbindungsschaft (8) eine Ausbauchung (8c) aufweist, die das Verankerungselement (4) und das Festlegeelement (6) in dem Formzustand der Klammer mit maximaler Spannung trennt.

11. Vorrichtung zum An-Ort-und-Stelle-Bringen einer Klammer gemäß einem der Ansprüche 1 bis 10, **dadurch gekennzeichnet, dass** sie das vorstehend genannte Rohr (51) und die vorstehend genannte Klammer (2) aufweist, wobei Letztere mit zu dem distalen Ende des Rohrs (51) gerichtetem Festlegeelement (6) und mit zu dem proximalen Ende des Rohrs (51) gerichtetem Verankerungselement (4) in das Rohr (51) eingesetzt wird; das Rohr (51) weist ein scharfes distales Ende (52) auf, das eine seitliche Öffnung aufweist, und ist mit einem Mittel (54) zum Zurückhalten des Verankerungselements (4) diesseits eines Kraftschwellenwertes versehen; die seitliche Öffnung ist derart bemessen, dass, wenn die Klammer (2) in das Rohr (51) in Richtung proximal-distal geschoben wird, ein Herausziehen des Festlegeelements (6) aus dem Rohr heraus möglich ist, und das Mittel (54) zum Zurückhalten ist derart positioniert, dass es das Zurückhalten des Verankerungselements (4) in dem Rohr gewährleistet, wenn das Festlegeelement (6) so aus dem Rohr herausgezogen wird; das Mittel (54) zum Zurückhalten ist derart beschaffen, dass es diesseits des genannten Kraftschwellenwertes das Zurückziehen der Klammer (2) mit dem Rohr (51) ermöglicht, und dass, sobald das Festlegeelement (6) in Anlage gegen das prothetische Stück (100) kommt und das prothetische Stück (100) gegen den anatomischen Träger (101) gedrückt wird, es einen zusätzlichen Zug auf das Rohr (51) erfordert, um das Verankerungselement (4) zu befreien, wodurch somit dem Verankerungselement (4) eine ausreichende Verankerungstiefe gewährleistet wird, um zu garantieren, dass das prothetische Stück (100) gut gegen den anatomischen Träger (101) angedrückt ist.

12. Vorrichtung nach Anspruch 11, **dadurch gekennzeichnet, dass** das Rohr (51) ein offenes und scharfes distales Ende (52) aufweist, und dass der distale Eindringabschnitt des Rohrs (51) diesseits des distalen Endes durchgehend und massiv ist, derart, dass es zumindest eine Klammer (2) in ihrem gespannten rohrförmigen Formzustand vollständig enthalten kann.

13. Vorrichtung nach Anspruch 11 oder 12, **dadurch gekennzeichnet, dass** der distale Eindringabschnitt der Nadel (51) eine Charge oder eine Mehrzahl an Klammern (2) aufweist, die Ende an Ende angeordnet sind, und zwar jede in ihrem gespannten rohrförmigen Formzustand.

14. Vorrichtung nach einem der Ansprüche 11 bis 13, **dadurch gekennzeichnet, dass** sie außer der Nadel einen Stößel (53) zum Schieben des proximalen Endes einer Klammer auf weist, die in ihrem gespannten rohrförmigen Formzustand in dem distalen Eindringabschnitt der Nadel angeordnet ist, wobei der Stößel dazu ausgelegt ist, in das Innere der Nadel einzudringen und sich im Inneren der Nadel frei zu verschieben, und ein proximales und äußeres Ende zu seiner mittelbaren oder unmittelbaren Betätigung aufweist.

15. Vorrichtung nach Anspruch 14, **dadurch gekennzeichnet, dass** das äußere und proximale Ende des Stößels (53) ein Betätigungsorgan aufweist.

16. Vorrichtung nach einem der Ansprüche 11 bis 15, **dadurch gekennzeichnet, dass** der distale Eindringabschnitt eine Charge oder eine Mehrzahl an Klammern (2) aufweist, die jeweils in ihrem gespannten rohrförmigen Zustand Ende an Ende angeordnet sind.

17. Vorrichtung nach einem der Ansprüche 14 bis 16, **dadurch gekennzeichnet, dass** sie ein Mittel zur stufenweisen Markierung des Vorrückens des Stößels (53) in das Innere der Nadel (51) aufweist.

18. Vorrichtung nach einem der Ansprüche 14 bis 17, **dadurch gekennzeichnet, dass** sie aufweist:
- einen Griff (70), an dem die Nadel (51) an einem distalen Ende des Griffs koaxial angebracht ist,
- einen Schlitten (74), der koaxial im Inneren des Griffs (70) frei translatorisch bewegbar angebracht ist, wobei der Schlitten eine Reihe von axialen Anschlägen (74a, 74b, 74c) in einer Anzahl aufweist, die um eins geringer ist als die Anzahl an Klammern (2), die in dem distalen Eindringabschnitt der Nadel (51) angeordnet sind, und die entlang der Achse des Handgriffs in einem selben Abstand, der dem Elementarhub des hiernach definierten Vorrückens des Stößels (53) entspricht, verteilt und getrennt sind,
- ein Schuborgan (80), das aus dem Griff herausragt, und das im Inneren des Griffs translatorisch beweglich auf der bezüglich der Nadel (51) anderen Seite des Schlittens angebracht ist,
- ein Rückstellmittel (82) zum Zurückstellen des Schlittens in eine Ruhe- oder Rückstellposition, in der das proximale Ende des Schlittens (74) und das distale Ende des Greiforgans (80) gegeneinander in Anschlag stehen,
- wobei der Stößel (53) distalseitig im Inneren der Nadel koaxial angeordnet und proximalseitig mit einem Gegenanschlag (90) fest verbunden ist,
- ein Antirückziehmittel (84) zwischen dem Griff (70) und dem Stößel (53), der die translatorische Bewegung des Stößels zur Vorderseite des Stiels (70) ermöglicht und seine Rückwärtsbewegung, stets bezüglich des Handgriffs, blockiert,
- wobei jeder der axialen Anschläge (74a, 74b, 74c) des Schlittens dazu ausgelegt ist, dem Gegenanschlag (90) auszuweichen, wenn der Schlitten nach hinten in seine Ruhestellung rückgestellt wird, wobei der Stößel bezüglich der translatorischen Bewegung durch das Antirückziehmittel blockiert ist.

19. Vorrichtung nach Anspruch nach Anspruch 18, **dadurch gekennzeichnet, dass** das Schuborgan (80) von dem Schlitten (74) verschieden ist oder mit dem Schlitten (74) ein Stück bildet.

20. Vorrichtung nach Anspruch 18, **dadurch gekennzeichnet, dass** jeder axiale Anschlag (74a, 74b, 74c), um dem Gegenanschlag (90) auszuweichen, aus einem oder mehreren radial nach außen elastischen Zähnen gebildet ist, oder dass jeder axiale Anschlag (74a, 74b, 74c) aus einer Sperrklinke gebildet und der Gegenanschlag aus zumindest einem radial nach innen elastischen Zahn gebildet ist.
